# EUROPEAN PATENT SPECIFICATION

(11) **EP 2 009 437 B1**
(45) Date of publication and mention of the grant of the patent: **18.08.2010**
(21) Application number: 07111044.9
(22) Date of filing: 26.06.2007
(51) Int. Cl.: G01N 33/14

(54) **Device and method for assaying a vinefication liquid**
Vorrichtung und Verfahren zum Testen einer Vinifikationsflüssigkeit
Dispositif et procédé pour l'analyse d'un liquide de vinification

(43) Date of publication of application: 31.12.2008
(73) Proprietor: FOSS Analytical A/S, 3400 Hilleroed (DK)
(72) Inventor: Juhl, Henrik Vilstrup, DK-4000, Roskilde (DK)
(74) Representative: Hilton, Charles Paul

(56) References cited:
- DE-B3-102005 027 601
- ES-A1- 2 051 167
- FR-A- 1 242 376
- FR-A1- 2 709 308
- GB-A- 2 305 935

## Description

### Background art

The present invention relates to a system for and a method of assaying alcoholic liquid vinefication products, from the fermented grape juice to the finally produced wine, by means of optical spectroscopy.

As used herein the term "optical" shall be taken to mean any or all of that portion of the electromagnetic spectrum that extends from ultraviolet to mid infra-red.

The actual taste of the produced wine, hence its quality, depends not only on the care with which the fermentation was carried out but also on the constituent compounds of the grape juice. Three dominant groups of compounds in the grape juice influencing the wine quality are phenolic compounds influencing flavour and colour, organic acids including malic, citric, and lactic acids influencing the flavour and mouth-feel and sugars influencing the flavour and the obtainable alcohol content.

However, the taste or the quality of a wine is dependent on a complex mixture of compounds, some of which are present in the basic grape juice, others of which are formed during the fermentation process and finally some which are added during the fermentation process, including sulphur dioxide. A great many of the compounds known to influence the quality of the wine product are present in amounts typically less than about 0.1 gram per litre (0.1 g/l), such as, free and bound sulphur dioxide; phenols; tannins; ethylacetate; ascorbic acid; methanol; citric acid; chimic acid; succinic acid; and anthocyanins, and other important constituents such as acetic acid are influenced by the signal to noise ratio of analytical methods, and the usefulness of a method will be improved by an improved signal to noise ratio, which can be obtained by increasing the concentration of the constituent analysed.

It is well known to provide optical spectroscopy based assay systems for one or both of the quantitative and the qualitative determination of components of a liquid vinefication product which affect the quality of the final wine product. One such system is disclosed in US 6885003 (MARC DUBERNET) 26.04.2005 . Here a broadband infrared Fourier transform interferometric optical spectrometer is employed in the assay of liquid vinefication products by means of chemometric analysis of spectral data obtained by the spectrometer. In this manner a wide variety of compounds may be detected and employed in the determination of a quality index for the sample.

Another known system for the assay of liquid vinefication products using optical spectrometers is disclosed in EP 1175603 B (CELOLTI ET AL) 30.01.2002 . Here a spectrometer is employed to assay grape juice by means of the chemometric analysis of spectral data obtained in the ultra-violet and visible regions of the electromagnetic spectrum.

The limit of detection of optical spectrometric instrumentation that is typically employed in such commercially available systems is of the order of 0.1 g/l. Many of the compounds that may affect the quality of the final wine product are therefore present in amounts just above or less than the limit of detection of the apparatus. Such compounds thus remain undetected or at least are detected with an associated large uncertainty (often quoted as a Standard Error of Prediction or SEP).

It is known to enhance the sensitivity of analytical methods based on optical spectrometers by adding a sample concentration step to the analytical method, and even to automate this step. A typical method for the sample concentration is the use of an extraction column, on which one or more of the compounds of interest are bound, and subsequently released as a concentrate by eluting with a suitable solvent such as ethyl acetate. One such example is given by C. CHILLA, D. A. GUILLÉN, et al. Automated on-line-solid-phase extraction-high-performance liquid chromatography-diode array detection of phenolic compounds in sherry wine . Journal of Chromatography A. 1996, vol.750, no.1-2, p.209-214. However such a method requires addition of a solvent and results in a relatively mechanically complex and expensive arrangement which is commercially undesirable and addition of a solvent is furthermore related to extra cost, and environmentally undesired.

According to the present invention there is provided a system for and a method of assaying a liquid vinefication product by utilizing optical spectrometric analysis and in which a freeze extraction process is employed to reduce the levels of water present in the sample to be assayed. By freeze distillation the effective concentration of analytes of interest in the sample is increased and hence the capability of an unmodified optical spectrometer to detect their presence is enhanced. Moreover, since water often produces interfering or 'background' spectral information in the regions of interest for the assay then the reduction of the amount of water also leads to a reduction in this background against which spectral information of interest is to be identified.

Preferably the amount of water extracted is determined, either from the extract or from the concentrate, and employed by the data processor in calculating back the content of the detected analytes of the original liquid vinefication product.

Usefully, the freeze distillation process involves freezing out water, preferably in a freezer and more preferably a freezer including an electro-thermal freezer element such as a peltier element. Freezing has the advantage that a relatively simple control of temperature ensures that the liquid phase of the sample has a specific ratio of ethanol to water and such electro-thermal freezers are generally compact in size and may be readily integrated in a housing together with the optical spectrometer. By arranging the freeze distillation unit in close physical proximity to the optical spectrometer transfer means, such as a flow pump and associated conduit, these may be connected to permit the automatic transfer of concentrated sample to the spectrometer for assay.

These and other advantages will be further clarified from a reading of the following description of exemplary embodiments of the present invention, made with reference to the drawings of the accompanying figures, of which:

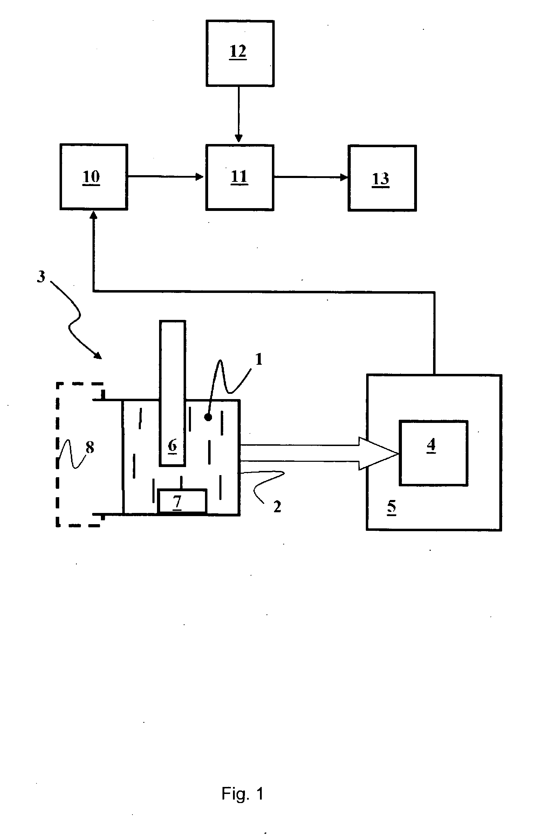

Fig. **1** is a schematic diagram an exemplary device according to the present invention.

Referring to Fig.**1**, a device for carrying out spectrometric assay of liquid vinefication products consists of a container **2** in which a liquid vinefication product **1** is positioned. One or two freezer units **3** is used to freeze and extract water as ice crystals. The non-frozen concentrated part of the product **1** (liquid distillate) is then transferred, as a sample for analysis, to a spectroscopic sample cell **4** of a spectrometer **5,** which records spectra **10** of the concentrated sample. The spectra **10** are subjected to a calibration **11** generated from a spectral database **12** to yield, by means of standard chemometric analysis techniques, an output **13** indicative of one or more desired components of interest in the sample, for example corresponding to a concentration of one or more desired components in the liquid vinefication product **1.**

The freezer unit **3** can be any device which can obtain a controlled temperature below -10°C, for example a thermoelectric device, or a vapour compression cycle freezer, with associated temperature control. Alternatively the freezer unit **3** may comprise means (not shown) for the addition of a cold material, such as liquid nitrogen or solid carbon dioxide to the liquid vinefication product **1.**

Usefully, as in the present embodiment, the freezer unit **3** may comprise a cooling element **6** removably locatable within a container **2** for the vinefication product **1.** Alternatively the container **2** may be placed inside a freezing volume of freezer unit **3.**

If the freezer unit **3** is of the former type then the preferred cooling element **6** should be a thermoelectric device, such as a peltier device, due to the compactness of such a design. Alternatively the liquid vinefication product **1** may be cooled by means of a heat conducting cooling element with one end connected to a cooling element and one end in the container **2,** submerged in the liquid **1.**

It will be apparent to a person skilled in the art that the choice of freezing methodology depends upon, amongst other things, the temperature desired, the volume of sample required, and the time of analysis desired. For small volumes of sample less energy transfer is required and accordingly a thermoelectric device may be preferred.

If a container **2** made of a material with poor heat conducting abilities is chosen then a design where the freezer unit **6** is positioned inside the container **2** is preferred.

Usefully the transfer of energy between the liquid **1** and the cooling element **6** or the wall of the container **2** may be increased by inducing a movement of the liquid sample relative to the container wall and/or the cooling element **6,** by vibration or oscillation of the container **2** and/or the cooling element **6.** Said vibration may be obtained by a vibrator **7** employing conventional mechanical or ultrasonic means.

The transfer of concentrated sample, in the form of a liquid distillate, from sample container **2** to spectroscopic sample cell **4** may be made in several ways. If the cooling element **6** is submerged in the liquid **1,** removing the element **6** from the container **2** at the time where the liquid **1** is partially frozen will remove the crystallised water from the liquid **1,** and leave a concentrated sample in the container **2.** Provided that at least a portion of the container **2** is formed of optically transparent material then the container **2** may thus have a double function as container **2** for the liquid vinefication product and as spectroscopic sample cell **4.**

Alternatively the concentrated sample may be transferred automatically by means of pumping or by opening a valve between container **2** and a spectroscopic sample cell **4** or manually by pouring the concentrated sample into the spectroscopic sample cell **4.**

If a design is used in which the container **2** is placed inside a freezing volume of the freezer unit **3** the container **2** may usefully be provided with a means for withholding the frozen fraction of the liquid in the container **2,** such as a restriction, a partial perforation of the container **2** or a perforated lid (broken construction **8),** or possibly to employ a syringe as sample container and separate the liquid fraction from the solid fraction of the slush by compression utilising the syringe piston.

The spectrometer **4** used may collect spectra **10** in the infrared, near infrared or ultraviolet/visual range. For wine analysis with focus on fermentation parameters, such as sugar content and acid balance, an infrared spectrometer is the preferred choice of spectrometer **4,** but for data related to colour and phenolic content ultraviolet and visual wavelength ranges may be preferred. A near infrared spectrometer may be preferred in the case where cost is important.

The spectra **10** are converted to results **13** such as concentrations by employing a calibration algorithm **11** such as partial least squares or artificial neural networks, calculated from a spectral database **12** with spectra and reference values recorded previously.

## Claims

1. A device for the spectrometric assay of liquid vinefication products (**1**) comprising a spectroscopic sample cell (**4**) for holding a sample to be analysed; an optical spectrometer (**5**) adapted to generate spectral information from the sample in the spectroscopic sample cell (**4**); a data processor (**10**) having access to a calibration formula (**11**) developed from a spectral database (**12**) linking optical spectral information to compounds of interest in the liquid vinefication product and configured to process the same to generate an output result (**13**) indicative of the presence of one or more compounds of interest in the liquid vinefication product (**1**) **characterized in that** the device further comprises a freezer unit (**3**) adapted for freeze distillation of the liquid vinefication product (**1**) to generate a liquid distillate as the sample to be analysed in the spectroscopic sample cell (**4**).

2. A device according to Claim 1 **characterised in that** the freezer unit (**3**) further comprises a container (**2**) for the liquid vinefication product **(1)** and a cooling element (**6**) removably locatable inside the container (**2**).

3. A device according to Claim 2 further **characterised in that** the cooling element (**6**) comprises a peltier cooler.

4. A device according to Claim 1 further **characterised in that** the freezer unit (**3**) is based on the addition of a cold substance such as liquid nitrogen or solid carbon dioxide.

5. A device according to Claim 1 further comprising a sample container (**2**) including a means (**8**) for separation of liquid concentrate and water crystals.

6. A device according to Claim 1, **characterised in that** there is further provided means (**7**) to effect relative motion between the liquid vinefication product (**1**) and the freezer unit (**3**).

7. A device according to any one of the claims above **characterised in that** data processor (**10**) is further adapted to determine the presence of the compounds of interest as an amount in the liquid vinefication product (**1**) by a calculation based on spectral information before and after freeze distillation.

8. A method of assaying a liquid vinefication product comprising steps of providing a sample from the liquid vinefication product, analysing the sample using an optical spectrometer and calculation of a quantity of a compound of interest contained by statistical means, **characterised in that** the step of providing a sample comprises freeze distillation of the liquid vinefication product to generate a liquid distillate having a reduced water content.

9. A method according to Claim 8 **characterised in that** there is provided an additional step of determining the amount of the compound of interest in the liquid vinefication product by a calculation based on spectral information obtained before and after freeze distillation.

## Patentansprüche

1. Eine Vorrichtung für die spektrometrische Untersuchung von flüssigen Vinifikationsprodukten (1), die eine spektroskopische Probenzelle (4) zum Halten einer zu analysierenden Probe; ein optisches Spektrometer (5), das angepasst ist, um von der Probe in der spektroskopischen Probenzelle (4) spektrale Informationen zu erzeugen; einen Datenprozessor (10), der Zugang zu einer Kalibrierungsformel (11), welche aus einer spektralen Datenbank (12) entwickelt ist, die optische spektrale Informationen mit Verbindungen von Interesse in dem flüssigen Vinifikationsprodukt verknüpft, aufweist und der konfiguriert ist, um dieselbe zu verarbeiten, um ein Ausgabeergebnis (13) zu erzeugen, das indikativ für das Vorhandensein von einer oder mehreren Verbindungen von Interesse in dem flüssigen Vinifikationsprodukt (1) ist, beinhaltet, **dadurch gekennzeichnet, dass** die Vorrichtung ferner eine Gefriereinheit (3) beinhaltet, die zur Gefrierdestillation des flüssigen Vinifikationsprodukts (1) angepasst ist, um ein flüssiges Destillat als die in der spektroskopischen Probenzelle (4) zu analysierende Probe zu erzeugen.

2. Vorrichtung gemäß Anspruch 1, **dadurch gekennzeichnet, dass** die Gefriereinheit (3) ferner einen Behälter (2) für das flüssige Vinifikationsprodukt (1) und ein Kühlelement (6), das entfernbar innerhalb des Behälters (2) lokalisierbar ist, beinhaltet.

3. Vorrichtung gemäß Anspruch 2, ferner **dadurch gekennzeichnet, dass** das Kühlelement (6) einen Peltier-Kühler beinhaltet.

4. Vorrichtung gemäß Anspruch 1, ferner **dadurch gekennzeichnet, dass** die Gefriereinheit (3) auf der Zugabe einer kalten Substanz wie etwa Flüssigstickstoffs oder festen Kohlendioxids basiert.

5. Vorrichtung gemäß Anspruch 1, die ferner einen Probenbehälter (2) einschließlich eines Mittels (8) zur Trennung von flüssigem Konzentrat und Wasserkristallen beinhaltet.

6. Vorrichtung gemäß Anspruch 1, **dadurch gekennzeichnet, dass** ferner ein Mittel (7) zum Bewirken von relativer Bewegung zwischen dem flüssigen Vinifikationsprodukt (1) und der Gefriereinheit (3) bereitgestellt wird.

7. Vorrichtung gemäß einem der obigen Ansprüche, **dadurch gekennzeichnet, dass** der Datenprozessor (10) ferner angepasst ist, um das Vorhandensein der Verbindungen von Interesse als eine Menge in dem flüssigen Vinifikationsprodukt (1) durch eine Berechnung, basierend auf spektralen Informationen vor und nach der Gefrierdestillation, zu bestimmen.

8. Ein Verfahren zur Untersuchung eines flüssigen Vinifikationsprodukts, das die Schritte des Bereitstellens einer Probe des flüssigen Vinifikationsprodukts, des Analysierens der Probe unter Verwendung eines optischen Spektrometers und der Berechnung einer Quantität einer enthaltenen Verbindung von Interesse durch statistische Mittel beinhaltet, **dadurch gekennzeichnet, dass** der Schritt des Bereitstellens einer Probe die Gefrierdestillation des flüssigen Vinifikationsprodukts beinhaltet, um ein flüssiges Destillat mit einem reduzierten Wassergehalt zu erzeugen.

9. Verfahren gemäß Anspruch 8, **dadurch gekennzeichnet, dass** ein zusätzlicher Schritt der Bestimmung der Menge der Verbindung von Interesse in dem flüssigen Vinifikationsprodukt durch eine Berechnung, basierend auf spektralen Informationen, die vor und nach der Gefrierdestillation erhalten werden, bereitgestellt wird.

## Revendications

1. Un dispositif pour le dosage spectrométrique de produits de vinification liquides (1) comprenant une cellule à échantillon spectroscopique (4) destinée à tenir un échantillon devant être analysé ; un spectromètre optique (5) adapté pour générer des informations spectrales provenant de l'échantillon dans la cellule à échantillon spectroscopique (4) ; un processeur de données (10) ayant accès à une formule d'étalonnage (11) développée à partir d'une base de données spectrale (12) associant des informations spectrales optiques à des composés d'intérêt dans le produit de vinification liquide et configuré pour traiter celles-ci afin de générer un résultat en sortie (13) indiquant la présence d'un ou de plusieurs composés d'intérêt dans le produit de vinification liquide (1) **caractérisé en ce que** le dispositif comprend en outre une unité formant congélateur (3) adaptée pour la cryodistillation du produit de vinification liquide (1) afin de générer un distillat liquide comme échantillon devant être analysé dans la cellule à échantillon spectroscopique (4).

2. Un dispositif selon la revendication 1 **caractérisé en ce que** l'unité formant congélateur (3) comprend en outre un récipient (2) destiné au produit de vinification liquide (1) et un élément de refroidissement (6) pouvant être placé à l'intérieur du récipient (2) de façon à pouvoir être retiré.

3. Un dispositif selon la revendication 2 **caractérisé en outre en ce que** l'élément de refroidissement (6) comprend un refroidisseur à effet Peltier.

4. Un dispositif selon la revendication 1 **caractérisé en outre en ce que** l'unité formant congélateur (3) est basée sur l'ajout d'une substance froide telle que de l'azote liquide ou du dioxyde de carbone solide.

5. Un dispositif selon la revendication 1 comprenant en outre un récipient à échantillon (2) comportant un moyen (8) destiné à la séparation de concentré liquide et de cristaux d'eau.

6. Un dispositif selon la revendication 1, **caractérisé en ce qu'**il est en outre prévu des moyens (7) pour effectuer un mouvement relatif entre le produit de vinification liquide (1) et l'unité formant congélateur (3).

7. Un dispositif selon n'importe laquelle des revendications ci-dessus **caractérisé en ce que** le processeur de données (10) est en outre adapté pour déterminer la présence des composés d'intérêt en tant que quantité dans le produit de vinification liquide (1) par un calcul basé sur des informations spectrales avant et après cryodistillation.

8. Une méthode pour le dosage d'un produit de vinification liquide comprenant des étapes consistant à fournir un échantillon prélevé sur le produit de vinification liquide, analyser l'échantillon à l'aide d'un spectromètre optique et calculer une quantité d'un composé d'intérêt contenu par des moyens statistiques, **caractérisée en ce que** l'étape consistant à fournir un échantillon comprend la cryodistillation du produit de vinification liquide afin de générer un distillat liquide ayant une teneur en eau réduite.

9. Une méthode selon la revendication 8 **caractérisée en ce qu'**il est prévu une étape supplémentaire consistant à déterminer la quantité du composé d'intérêt dans le produit de vinification liquide par un calcul basé sur des informations spectrales obtenues avant et après cryodistillation.
